# EUROPEAN PATENT SPECIFICATION

(11) **EP 0 783 344 B1**
(45) Date of publication and mention of the grant of the patent: **19.05.1999**
(21) Application number: 95936231.0
(22) Date of filing: 29.09.1995
(51) Int. Cl.: A61N 1/30

(54) **IONTOPHORETIC DRUG DELIVERY DEVICE HAVING IMPROVED CONTROLLER AND PATCH**
VORRICHTUNG ZUR IONTOPHORETISCHEN VERABREICHUNG VON MEDIKAMENTEN MIT VERBESSERTER STEUEREINRICHTUNG UND KISSENELEKTRODE
DISPOSITIF D'ADMINISTRATION D'UN MEDICAMENT PAR IONOPHORESE, AYANT UN TIMBRE ET UN SYSTEME DE COMMANDE AMELIORES

(30) Priority: 30.09.1994 US 315377; 30.09.1994 US 315372
(43) Date of publication of application: 16.07.1997
(73) Proprietor: Becton, Dickinson and Company, Franklin Lakes, New Jersey 07417-1880 (US)
(72) Inventor: FLOWER, Ronald , J., Vernon, NJ 07461 (US)
(74) Representative: Ruffles, Graham Keith
(86) International application number: US9512633
(87) International publication number: WO9610440

(56) References cited:
- EP-A- 0 461 680
- DE-A- 4 028 125
- US-A- 4 942 883
- US-A- 5 254 081

## Description

### BACKGROUND OF THE INVENTION

### 1. Field of the Invention

The present invention relates to iontophoretic drug delivery systems for transdermally delivering a drug or medicine to a patient, and more particularly relates to iontophoretic delivery systems having a number of features which improve usability and traceability of the iontophoretic drug delivery device, and the compatibility of a reusable iontophoretic controller with an iontophoretic patch.

### 2. Background of the Related Art

Iontophoresis may be defined as the introduction, by means of an electric current of ions of soluble salts, into the tissues of the body for therapeutic purposes. Iontophoretic devices, have, in recent years, become an increasingly important means of administering therapeutic agents. Such systems offer advantages clearly not achievable by any other methods of administration, such as by ingestion or by injection through the skin.

Presently, known iontophoretic devices use at least two electrodes, which are in contact with a portion of a patient's body. A first electrode, generally called the active electrode, delivers the ionic substance or drug into the body by iontophoresis. The second electrode, generally called the counter electrode, closes an electrical circuit that includes the first electrode and the patient's body. Generally, the circuit includes a source of electrical energy, such as a battery. The ionic substance to be driven into the body may be either positively charged or negatively charged. In the case of a positively charged ionic substance, the anode of the iontophoretic device becomes the active electrode to complete the circuit. Alternatively, if the ionic substance to be iontophoretically delivered is negatively charged, the cathode will be the active electrode and the anode will be the counter electrode.

EP-A-461,680 describes a programmable control and mounting system for a transdermal drug applicator. For application to a living body for the delivery of at least one drug through the skin into the bloodstream an applicator includes at least one drug reservoir containing the drug for delivering same through the skin by physico/chemical mass transfer. A mounting structure is removably mounted to the body for holding the applicator to the skin , with the applicator removably connected to the mounting structure. A power supply for the applicator and a circuit transmitting electrical power from the power supply to the applicator is disclosed, wherein an electric circuit is created between the applicator and the skin. The device includes a computer with the mounting structure for receiving programmed instructions relative to the drug, and transmitting signals relative to the drug and the programmed instructions to the power supply for regulating the generation of power and delivery of drug through the skin.

One type of iontophoretic drug delivery device includes a separate, reusable controller, which can be removably, electrically coupled to a patch containing the therapeutic agent. The controller includes the electronics which control the amount and duration of current applied to the patch. Delivery of a drug to the patient may be accomplished at a constant rate over a long period of time or, alternatively, at periodic intervals. Thus, it may be necessary for the drug-containing patch to be maintained in contact with the patient's skin for a long period of time, either for continuous drug delivery, or to permit frequent interval delivery over a period of time.

In situations where the periodic delivery of the medicament is indicated, there is no need to maintain the source of electric current connected to the patch between doses. While the unobtrusive medicament-containing patch may remain attached to the patient, removability of the current source would permit the patient to be free from the cumbersome connection to the current source between doses. At such time as iontophoretic drug delivery is once again necessary, the medicament-containing patch attached to the patient's skin may be reconnected to the current source. Since the current source generally includes a battery, it would be helpful to know the number of times the controller has been used as well as the duration of the use. In this manner, one can ensure that the controller has sufficient energy to transdermally deliver the necessary dosage of medicament to the patient.

In situations where the iontophoretic device is applied by the patient himself, it would be helpful for health-care professionals to be able to determine if the patient has actually received the medication. For example, it would be beneficial if the controller could communicate with the health-care professional to provide proof that it has actually delivered the medication to the patient.

As previously noted, it may be necessary to use an iontophoretic drug delivery device over an extended period of time (i.e., longer than 24 hours) to delivery the necessary dosage of drug. As the length of delivery time increases, there is a need to develop small, unobtrusive iontophoretic delivery devices which can be easily worn on the skin under clothing.

In addition to the need for developing smaller iontophoretic devices, there is a need to reduce the cost of these devices in order to make them more competitive with conventional forms of therapy such as pills and subcutaneous injections. One manner of improving cost effectiveness is to have a reusable controller which includes the costly electronics that provides the current to drive the patch.

A recent development in iontophoretic devices is the use of a separate, reusable controller which is removably, electrically coupled to a patch containing the therapeutic agent or drug. The reusable controller includes a power source, such as a battery, and electronics which can control the amount of current applied to the patch as well as the amount of time current is to be applied. Such a device is described in United States Patent No. 4,942,883 ('883 patent). The iontophoretic device includes a power supply source and a microprocessor control system. The housing of the power supply source and microprocessor are arranged so that a carrier or patch containing a drug to be delivered to a patient can be attached to an open underside of the housing. The '883 patent teaches that the dosage rate (drug delivery time) and other required parameters for the known dosage to be administered by a certain patch are entered into the microprocessor control system by the user through the use of programmable pads or by a card or tape containing such information. A concern with this type of arrangement, a two component system having a reusable controller, is that the controller may be inadvertently used with an incompatible patch.

More specifically, if an error is made in programming the dosage rate or other dosage parameters, a patient may be severely harmed. For example, the patient may be burned by excessive current applied to the patch or the patient may be harmed by an excessive amount of drug being driven into the patient's body. Alternatively, if not enough drug is being delivered to the patient, the patient's condition may worsen. The prior art provides no safety measures to ensure that a reusable controller is utilized with a compatible patch to provide proper drug dosages and time periods of dosages. Therefore, there is a need for a system that verifies the compatibility of controllers and patches.

Yet another factor in the design of iontophoretic devices is to make the device cost efficient. In order for iontophoretic devices to be cost effective and competitive with conventional forms of therapy such as pills and subcutaneous injections, the cost of each component and feature must be kept to a minimum while ensuring the safety and health of the patient using the device. Accordingly, there is a need to keep the cost of the safety measures low while ensuring proper administration of a medicament.

It is an object of the present invention to provide an improved iontophoretic drug delivery device having a reusable controller which can record the number of times the controller has been used.

It is another object of the present invention to provide an improved iontophoretic drug delivery device having a controller which includes a clock for timing a useful life of the controller.

It is a further object of the present invention to provide an iontophoretic drug delivery device having a unique serial number embedded in the controller electronics for purposes of traceability and security.

It is yet another object of the present invention to provide an iontophoretic drug delivery device having a controller which is capable of recording dates, times and/or duration of usage and provide the resulting information to a health-care professional to evaluate patient compliance in receiving prescribed medication.

It is still another object of the present invention to provide an improved iontophoretic drug delivery device having a controller which includes an LED indicator. The LED indicator is capable of sending information from the controller to a health-care professional. Additionally, the LED can act as a photo receiver to receive information to thereby instruct the controller.

It is yet another object of the present invention to provide an iontophoretic device having a reusable controller which includes means to ensure that the patch and controller are compatible prior to any medicament being transdermally administered.

It is still another object of the present invention to provide a cost efficient iontophoretic device having proper safety measures to ensure compatibility of the controller with the medicament containing patch.

### SUMMARY OF INVENTION

The present invention provides an iontophoretic drug delivery device which includes a medicament-containing disposable patch removably attachable to the skin of the patient for transdermal delivery of an ionized medicament and a reusable controller removably, electrically connectable to said patch, the controller providing sufficient energy to said patch to drive the ionized medicament into the skin of a patient, characterised in that the controller includes means for detecting a number of times the controller is used.

In accordance with one embodiment of the present invention, the iontophoretic drug delivery device includes a medicament-containing disposable patch and a reusable controller selectively connectable to the patch. The patch is removably attachable to the skin of the patient for transdermal delivery of ionized medicament. The controller provides an energy source that powers the patch to drive the ionised medicament transcutaneously to the patient. The controller further includes means for sensing a number of times the controller is used. In this manner, the useful life of the iontophoretic controller can be tracked. In order to accomplish this task, the iontophoretic drug delivery device includes a microprocessor which counts the number of times the controller has been applied. Additionally, the microprocessor renders the controller unusable when a specific number of applications have been sensed to ensure that sufficient energy remains in the power source to drive the ionized medicament transcutaneously to the patient. The controller may further include a display for displaying the number of times the controller has been used and for indicating whether a useful life of the controller has expired.

In an alternative embodiment, the iontophoretic drug delivery device further includes a clock, which can be started at the date of manufacture, for timing a useful life of the controller. For example, the power source may have a life of approximately 5 years. Accordingly, when the clock has timed 5 years from the date of manufacture, the controller is no longer usable and should be discarded. The controller may include indication means to indicate that the useful life has expired and, additionally, means to render the controller unusable when the predetermined time period has expired.

In an alternative embodiment of the present invention, the iontophoretic drug delivery device further includes means for recording a date, time and/or duration of usage by the patient. This stored information can be transmitted to a health-care professional to evaluate patient compliance in receiving medication as prescribed. The recordation means includes a microprocessor to record the information and means for transmitting the stored information from the microprocessor to the health-care professional. The transmitting means may take any known form such as a serial port of the microprocessor or an LED which can act as a transmitter and photo receiver for receiving instructions from the microprocessor. The controller in accordance with the present invention having the recording feature, provides a fail-safe drug delivery system, so that the health-care professional can determine if the patient is actually receiving the medication prescribed. For example, the controller can inform the health-care professional that either it never delivered current to the patch or can provide proof that medication was delivered at a particular time, and on a particular date, so that the health-care professional can be assured that the patient received the medication as prescribed.

In an alternative embodiment of the present invention, the iontophoretic drug delivery device may also include a unique serial number stored in the read only memory (ROM) of the microprocessor. The purpose of the unique serial number is for traceability or tracking of the controllers. Accordingly, the controller can be tracked to determine which patient has a particular controller. Additionally, a physician or health-care professional may check the serial number to determine if a wrong patient is using the controller. Furthermore, the unique controller serial number may be used as a security device. More particularly, the patch may also include a unique serial number which can be read by the controller. A microprocessor within the controller compares the serial number of the patch with the serial number of the controller to ensure compatibility of the controller to the patch. It is important when using reusable controllers to ensure that the proper amount of current for the proper amount of time is applied to the patch to drive the specific medicament within the patch transcutaneously to the patient.

In accordance with another form of the present invention, the iontophoretic device also includes means for electrically matching the controller to the patch. In this manner, the iontophoretic device prevents current from the controller from being applied to the patch if the controller and patch are determined to be incompatible.

In order to determine if the controller and patch are compatible, the iontophoretic drug delivery device includes electronic means located on either the patch, the controller or both, which enables the controller to determine its compatibility with the patch. More specifically, in one embodiment of the present invention, both the patch and controller include a resistive network, which based upon the ratios of the resistances of the controller and patch network circuits, the controller can determine compatibility with the patch to which it is connected. Compatibility of the patch and controller is defined as the controller providing a proper dosage rate (a specific current over a specified period of time) for the medicament to be transdermally driven from the patch into the patient's body. Accordingly, if the controller and patch are deemed by the controller to be incompatible, the controller will not permit current to flow to the patch. Thus, the system ensures the safety of the user, by only permitting compatible controllers and patches to be utilized by the patient.

The controller preferably includes a microprocessor to determine compatibility of the controller with the patch. The microprocessor can be preprogrammed to deliver the correct dosage rate for a specific drug or therapeutic agent to be iontophoretically delivered to the patient. Alternatively, by identifying a patch, the controller can program itself to deliver the correct dosage.

In an alternative embodiment of the present invention, the safety measure for compatibility of the controller and patch includes a serial number located on the patch and the controller including means for reading the serial number of the patch. If the serial number read by the controller corresponds to a serial number programmed into the microprocessor of the controller, the controller determines that the patch and controller are compatible and will provide current to transdermally drive the medicament into the body of the patient.

The serial number may be formed by an integrated chip embedded in the patch. Additionally, the microprocessor in the controller may poll the patch serial number and compare it to a look-up table in the microprocessor for patch compatibility. If the patch is determined to be incompatible, no current will be permitted to be applied to the patch by the controller.

The present invention is also directed to a method for ensuring proper iontophoretic drug using an iontophoretic device dosage including a patch containing a drug and a reusable controller, comprising the steps of:
electrically comparing an identification of the controller to an identification of the patch to determine compatibility of the controller with the patch and permitting current to flow from the controller to the patch if the identification of the controller is determined to be compatible with the identification of the patch.

A preferred embodiment of the iontophoretic drug delivery device, as well as other embodiments, objects, features and advantages of this invention, will be apparent from the following detailed description of illustrative embodiments thereof, which is to be read in connection with the accompanying drawings.

### BRIEF DESCRIPTION OF THE DRAWINGS

Figure 1 is a schematic illustration of an iontophoretic drug delivery device formed in accordance with the present invention.
Figure 2 is a perspective view of an iontophoretic patch and a reusable controller of the present invention shown prior to connection.
Figure 3 is a perspective view of an iontophoretic patch and controller of the present invention shown after connection.
Figure 4 is a perspective view of the iontophoretic drug delivery device of the present invention attached to the skin of the patient.
Figure 5 is a block diagram of an iontophoretic device having a microprocessor in accordance with one form of the present invention.
Figure 6 is a schematic drawing of a patch illustrating a resistive network of the present invention.
Figure 7 is an illustration of a preferred patch resistive network of the present invention shown within the dashed outline (60) in Figure 4.
Figure 8 is a schematic design of the controller resistive network coupled to the patch resistive network of the present invention.
Figure 9 is an alternative circuit schematic of the controller circuit and patch circuit to ensure compatibility according to the present invention.

### DETAILED DESCRIPTION OF THE PREFERRED EMBODIMENT

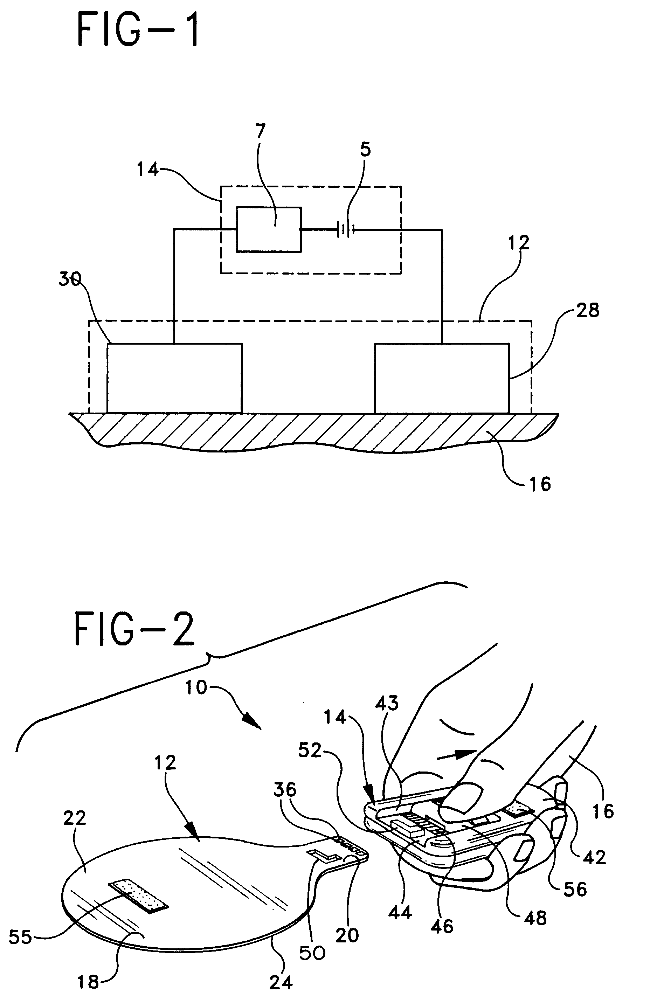

Referring to Figure 1, an iontophoretic drug delivery device 10 in accordance with the present invention includes a controller 14 electrically connected to a patch 12, which is coupled to the skin of a patient 16. The patch includes an active electrode assembly 28 and a counter electrode assembly 30. If a positively charged medicament is to be delivered to the patient's skin 16, the medicament would be positioned in the active electrode assembly. As shown in Figure 1, the iontophoretic drug delivery device also includes a controller 14 having a power supply 5 and a control circuit 7. The controller 14 is coupled to the patch 12 using well known means, for example, by printed flexible circuits, metal foils, wires, tabs or electrically conductive adhesives. The power supply 5 in combination with the electrode assemblies 28 and 30 and the patient's body 16 completes the circuit and generates an electric field across the body surface or skin to which the iontophoretic device is applied. The electric field generated by the power supply 5 causes the medicament in the electrode assembly 28 to be delivered into the body of the patient by the process of iontophoresis.

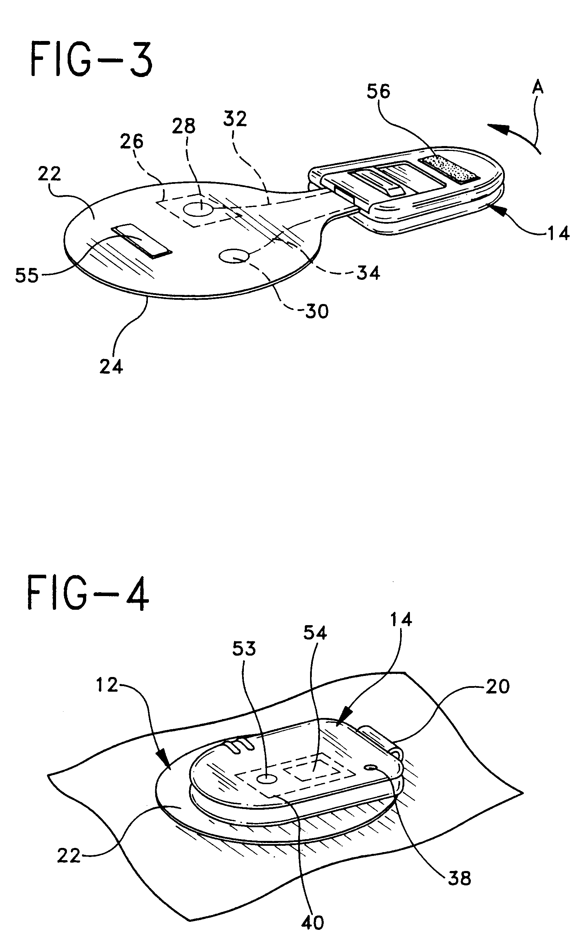

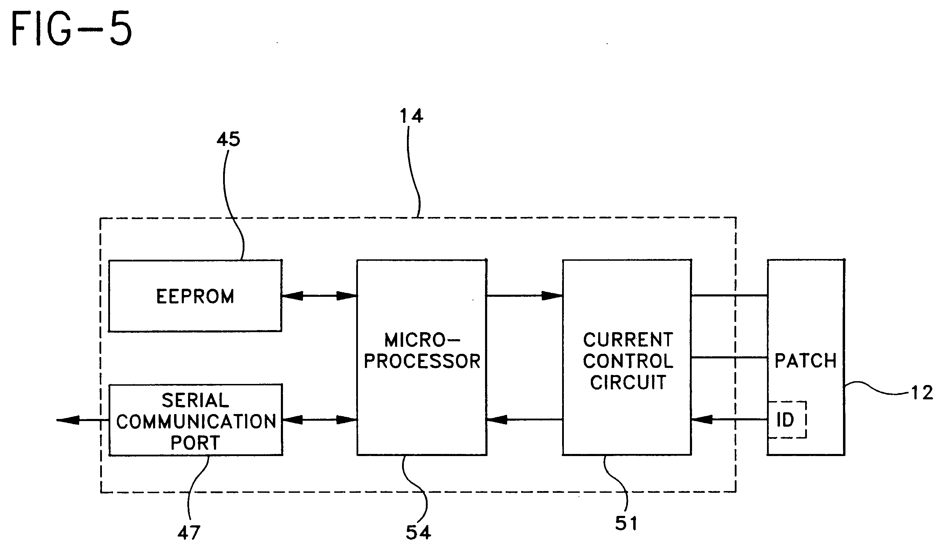

Referring now to Figures 2 and 3, an iontophoretic drug delivery device 10 including patch 12 and controller 14 of the present invention is shown. Patch 12 is a generally planar flexible member formed of biocompatible material. Patch 12 may be formed of woven or non-woven textiles or polymers or may be any other construction well known in the art. Patch 12 is preferably adhesively supported on the skin 16 of the patient (Fig. 4). Patch 12 includes an enlarged patch body 18 and an extending narrow tab 20. Patch body 18 includes opposed planar surfaces 22 and 24. Planar surface 24 is disposed for skin contact and includes a drug (i.e., medicament) reservoir 26 (shown in phantom in Figure 3) which contains an ionic drug typically in a gel form. While reservoir 26 is shown, any other known iontophoretic drug reservoir structure for placing a medicament in contact with the skin in an iontophoretic patch may be employed.

Skin contacting surface 24 further includes a pair of spaced apart electrodes 28 and 30 (shown in Figure 3). Each of electrodes 28 and 30 are positioned to be in contact with the skin once the patch 12 is secured, as shown in Figure 4. The positioning of electrodes 28 and 30 is such that an electrical current path is established between electrodes 28 and 30 through the skin of the patient. Electrode 28 is also placed in conductive contact with reservoir 26 in a manner well-known in the iontophoretic delivery art. A direct current source may be connected between the electrodes 28 and 30 such that electrode 28 in contact with reservoir 26 assumes the same charge as the ionised drug contained in reservoir 26. Under the influence of electrical current passing from the electrode 28 to electrode 30 through the skin, the drug contained in reservoir 26 is transcutaneously delivered.

Referring to Figure 3, electric current is supplied from the controller 14 to electrodes 28 and 30 on the patch via electrical traces 32 and 34. Each of traces 32 and 34 may be one or more conductive paths extending from electrodes 28 and 30 to exposed conductive pads 36 (shown in Figure 2) positioned on a marginal edge of the patch tab 20. As described in further detail below, pads 36 are positioned for electrical connection to the controller 14, which provides a source of electrical current.

Referring again to Figure 3, controller 14 houses electronic components 40 (shown in Figure 4) that control the supply of electric current to electrodes 28 and 30. As is known in the art, electrical components 40 may include a source of electrical power such as a battery 53 and additional electronic components, such as a microprocessor 54, used to send a controlled electrical current to electrodes 28 and 30.

As illustrated in Figures 2 and 3, controller 14 includes a controller housing 42 which is generally rectangular in shape and includes an open front end 44 which accommodates tab 20 of patch 12. Housing 42 further accommodates a connection array 46 adjacent electronic components 40 (Figure 4). The connection array 46 and electronic components are preferably mounted to a common printed circuit board (not shown). Connection array 46 may include plural electrical terminals in electrical connection with electronic components 40 and which are connectable to pads 36 of tab 20. In the present illustrative embodiment, connection array 46 is an electrical connection device having plural spaced-apart, exposed conductive surfaces separated by an insulating material. It may be appreciated that any suitable electrical interconnection device may be employed in accordance with the present invention.

Housing 42 further includes a cover 48 which is used to close the open front end 44 of housing 42. Cover 48 is slidably, captively retained on an upper wall 43 of housing 42. As shown in Figure 2, cover 48 may be manually moved under thumb actuation to an open position exposing connection array 46 for electrical connection with pads 36 of tab 20. Cover 48 may be moved to a closed position shown in Figure 3, covering connection array 46. With cover 48 in an open position, patch 12 may be connected to controller 14.

In order to assure accurate alignment of pads 36 of tab 20 with the connection array 46 supported within housing 42, tab 20 is keyed to housing 42. Tab 20 includes an opening 50 which is designed to fit over an upwardly extending post 52. Opening 50 and post 52 are of similar shape so as to provide keyed accommodation of tab 20 and post 52. Post 52 extends upwardly from a bottom wall 45 of housing 42 adjacent the open front end 44. Post 52 is centrally located adjacent connection array 46 to accommodate tab 20 and positionally confine tab 20 within housing 44. The key structure included on both opening 50 and post 52 prevents incorrect positioning of patch 12 with respect to controller 14. In the present embodiment, both opening 50 and post 52 have a generally L-shaped cross-section, however, any other mating shape which would prevent incorrect alignment may be employed.

Referring again to Figure 3, patch 12 and controller 14 include attachment means for permitting the releasable support of controller 14 on patch 12 after interconnection between pads 36 and connective array 46 is established. Surface 22, which is opposed to skin-engaging surface 24 of patch 12, and the upper surface of housing wall 43 include cooperating fastening elements 55 and 56 thereon. In the present illustrative embodiment, the cooperative fastening elements include conventional hook and loop fasteners of the type sold under the trademark VELCRO. Any other cooperating type fasteners may be employed to achieve the same objective. One cooperating fastening element 55 is secured adhesively or otherwise to patch 12 on surface 22 while the other cooperating fastening member 56 is secured by adhesive or otherwise to the upper surface of wall 43 of housing 42. As described in further detail below, attachment of the mating hook and loop fasteners 55 or 56 provide removable support for controller 14 on patch 12. It may be appreciated by those skilled in the art that the patch and controller may take any known form. The only requirement is that the patch be capable of being physically and electrically connected to the controller.

Having described one embodiment of iontophoretic drug delivery device 10 of the present invention, its operation is described below.

Patch 12 may be adhesively secured to the skin 16 of the patient. Surface 24 of patch 12 is placed in intimate contact with the skin 16 so that electrodes 28 and 30, as well as drug containing reservoir 26, are supported in intimate contact with the skin 16. In order to iontophoretically deliver the medicament from reservoir 26 transcutaneously through the skin 16, reusable controller 14 is electrically connected to patch 12. Housing 42 is slipped over extending tab 20 of patch 12 so that opening 50 in tab 20 is seated over upwardly extending post 52 of housing 42. Proper planar orientation is assured between patch 12 and controller 14 due to the key matability between opening 50 and post 52. As controller 14 is designed to be maintained in electrical connection with patch 12 during iontophoretic delivery of the drug contained in reservoir 16, controller 14 may be fastened to patch 12 so that it will be conveniently retained on the skin of the patient.

As shown in Figure 3, once patch 12 is connected to controller 14, the controller may be flipped up in the direction of arrow A so that the mating hook and loop fasteners 55 and 56 engage each other to removably fasten controller 14 to patch 12 as shown in Figure 4. The controller 14 is comfortably retained on the skin of the patient during iontophoretic drug delivery. At such time as a particular application of the drug is completed, the controller may be removed by separating the mating hook and loop fasteners 55 and 56. The controller may be disconnected and placed aside until the next administration of the drug is needed. The patch 12 may remain on the skin of the patient, eliminating the need for frequent replacement of the patch.

As previously indicated, when using a reusable controller, it would be advantageous to have means for counting the number to times the controller has been used. Since the controller includes the electronics and more particularly, the power source, the controller can only be used a specific number of times before the battery power is depleted. For example, a controller which is specifically programmed to provide current to a patch administering a specific medicament may need to operate at a certain current level for two hours in order to deliver the proper dosage. Under the conditions, the life expectancy of the controller may hypothetically be calculated to be 200 applications. In this situation, the controller preferably includes means for counting the number of applications and, when the maximum number has been counted, the controller includes a means for indicating it is no longer usable.

In the preferred embodiment of the present invention, the reusable controller includes electronics 40 (Figure 4) that control the current applied to a patch. The controller electronics include a battery 42 and a microprocessor 44. Other suitable electronics may be mounted on a printed circuit board incorporated within the controller housing. Among its other functions, the microprocessor 44 detects each time the controller is used and maintains a running total. Once, the microprocessor counts a specific number of applications, (i.e. 200) the microprocessor disables the controller from further applications. In this way, it can be assured that the controller has sufficient energy to drive the medicament from the patch to the patient's body so the patient will receive a proper dosage. The controller preferably includes means to indicate that the useful life of the controller has been exhausted. For example, referring to Figure 4, the controller may include a visual (or audio) indicator, such as an LED or LCD 38 which will be illuminated or turned off when the controller is no longer available.

In an alternate embodiment, the useful life of the reusable controller may be stored by a clock in the controller microprocessor. For example, the controller power source may have a calculated life expectancy, based upon the preprogrammed power requirements and shelf life of the battery, to drive a series of patches for approximately five years. The clock may be started at the time of manufacture of the controller. When the microprocessor has determined that the preprogrammed amount of continuous time from manufacture has expired, the controller will be rendered unusable and may be discarded or returned to the manufacturer. The controller may include similar indication means, such as an LED, to visually indicate to the user that the controller should no longer be used. Such a technique would be analogous to the expiration date on a bottle of pills. The manufaturer can thus be assured that an out-of-date controller can be rendered unusable, and this safety technique may satisfy some of the product liability concerns of the manufacturer.

The indicator 38 may be coupled to a driver circuit (not shown) which is responsive to a signal provided by the microprocessor 54. The microprocessor 54 will compare a running total of uses of the controller with a predetermined value and, for example, provide a signal to the indicator driver circuit that will cause the driver circuit to turn the indicator on or off. In this way, the patient will know that the controller can no longer be used and a new controller must be purchased.

When using an iontophoretic drug delivery device having a reusable controller, it would also be advantageous to identify which patient has a particular controller. Such a tracking system may include a unique serial number embedded in the controller. Referring to Figure 5, the unique serial number or prescription can be embedded in the read only memory (ROM) of the microprocessor. One method would be to permanently store the unique serial number in E²PROM at the time of manufacture or at the physician's office or pharmacy. The unique serial number can be read through a serial communication port by a health-care professional to determine the identification of the controller for tracking upon distribution. In Figure 5, the controller 14 is electrically connected to the patch 12. The controller includes a current control circuit 51, a microprocessor 54, an E²PROM 45 and a serial communication port 47. The traceability of the reusable controller is significantly enhanced by the readable unique serial number. Accordingly, a doctor or outside agency will be able to check the serial number to ensure that the patient has received the correct controller.

Additionally, the unique controller serial number may be used as a security device to ensure that the proper patch has been connected to the proper controller. An identification of the patch may be read into the microprocessor of the controller and compared to the unique controller serial number. If the microprocessor determines that the patch and controller, based upon its identification, are incompatible, the microprocessor will not allow current to be applied to the patch.

As well as traceability of the controllers, it would be advantageous for health-care professionals to have the ability to determine if the patient is receiving the desired dosage of medication. The controller of the present invention advantageously includes a microprocessor having an E²PROM having a sufficient memory to be able to record the date, time and/or duration of usage. The stored information may be played back by a technician or health-care professional. This becomes extremely important in monitoring a patient who is iontophoretically administering the drug away from a clinical setting. Using known electronics and microprocessor, such as Microchip PIC 16C71-04/50, manufactured by Microchip Technology, Inc. of Chandler, Arizona, the controller voltage, current, and duration of use can be recorded and stored for subsequent playback. Referring to Figure 5, the stored information may be played back through the serial communications port 47 of the controller 14. The recorded information can provide the necessary information to ensure that a proper dosage of medicament has been administered to the patient.

In an alternative embodiment, the controller includes an LED 38 (Figure 4) which can send and receive information to instruct the microprocessor 54. The LED is electrically coupled to the microprocessor so that information received and stored by the microprocessor can be transmitted via the LED to a technician or health-care professional. The stored data may be transmitted by the LED to a computer and displayed using any known means such as the display of the computer. Additionally, during the off time of the LED, the LED can be used as a photo-receiver for providing instructions or programming to the microprocessor in the controller. Accordingly, the improved iontophoretic drug delivery controller allows for two-way communication to receive data accumulated by the controller during use and to provide altered or new instructions to the controller without disassembly. The circuitry for performing the transmitting and receiving using LED's is well known to those skilled in the art and any such known circuitry may be used to accomplish this feature.

The present invention offers many features that are needed in the administration of medicaments by the use of iontophoretic devices. The improved controller of the present invention is capable of recording important information and playing the information back to health-care professionals. Additionally, the controller and patch design include important safety features to ensure the patient's safety misapplication of medicaments or excessive current to the patient.

The iontophoretic drug delivery device of the present invention is also specifically designed to ensure compatibility of the controller with the patch to which it is connected. This is an important feature when utilizing a reusable controller to avoid supplying an incorrect amount of energy to the patch and possibly harm the patient. Different medicaments require different rates of delivery and dosage. Additionally, medicaments having lower pluralities or higher molecular weights require a higher current to be delivered through the skin. The controller may also be specifically programmed to deliver either an anionic drug or a cationic drug. Thus, compatibility of the controller to the patch is very important. Accordingly, in the present invention, the controller, patch or both includes a means for determining whether the controller is compatible with the patch. If it is determined that the patch and controller are incompatible, the controller prevents power from being supplied to the patch. In an alternative embodiment the controller, after identifying a particular patch, may reprogram itself to suit the particular patch characteristics.

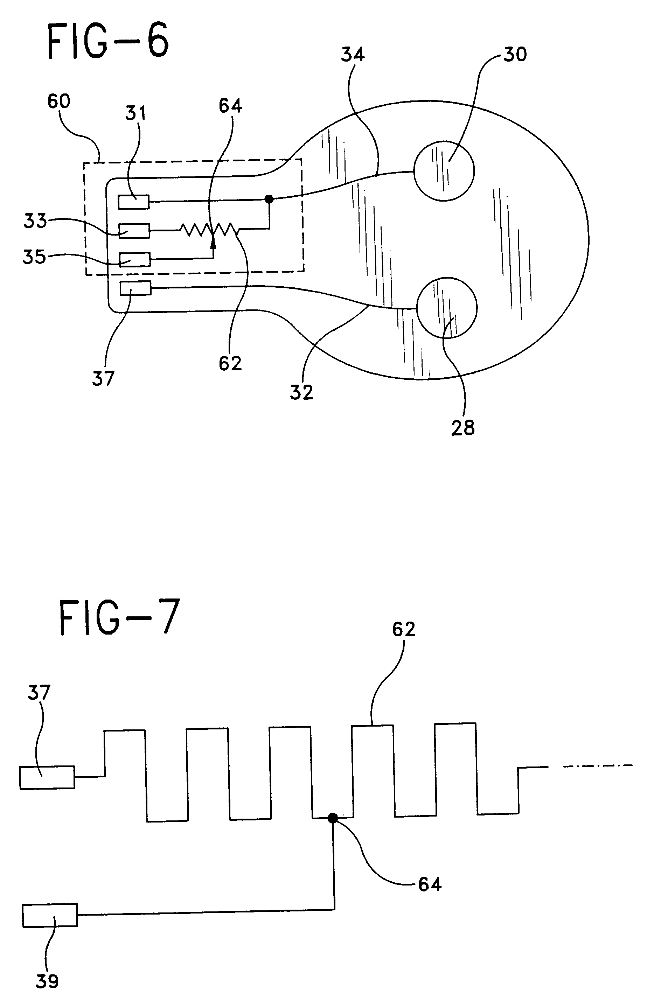

Referring to Figure 6, a patch including a resistive network 60 formed in accordance with the present invention is illustrated. In the preferred embodiment, the patch includes at least four connection tabs 31, 33, 35, 37. Two connection tabs 33, 35 are coupled via electrical lines 32, 34 to the patch electrodes 28, 30. It is preferable that the electrical lines 32, 34 be fabricated from a material having a low impedance. The resistive network 60 preferably comprises a resistor 62 which is preferably formed from an elongated resistive material which is tapped at a desired point along its length. The resistor 62 is coupled at one end to a connection tab 33 and its other end is coupled to electrical trace 34 which is in turn connected to the anode electrode of the patch. The tap 64 for the elongated resistor 62 is coupled at its other end to a separate connection tab 35. The resistive network 60 of the patch provides a unique resistive ratio to the patch depending upon the location of the tap in the elongated resistive material.

The elongated resistive material forming patch resistor 62 may take any known form. For space constraints, the elongated conductive material may take on a sepentine shape, such as the square wave illustrated by Figure 7, to increase the total resistance in the confined space available on the patch. Any periodically spaced "wave" may be conveniently tapped. The square wave shape and tapped "waves" will conveniently provide a visual indiction of the ratio of the resistances on opposite sides of the tap.

A common manufacturing process can be used to produce the patches, and the unique resistive ratio for the different patches is formed by tapping the different sections of the patch resistor 62. The resistor may be fabricated by silk screen printing or transfer process or by painting on the backing of the material of the patch. Preferably the electrical traces 32,34 are fabricated from a silver ink or paint for best conductivity and the resistor is fabricated from a high impedance material such as carbon or graphite paint or ink. The paint or silver ink, for example, preferably is homogeneousin content and in cross-sectional area along its length to provide a constant resistance per unit length. In this way, it is assured that the ratio of the resistances on opposite sides of the tap is predictable irrespective of the total resistance of the elongated resistive material from which resistor 62 is formed.

One advantage of this manufacturing process is that the patch leads or conductors, including traces 32 and 34 may be formed at the same time and in the same manner as the resistance network 60. Both may be formed on the patch substrate by the silk screening or ink painting process. The leads and the resistors of the network may be formed from the same material. The leads connecting to the network may be of a relatively short length and a wide cross-section so that they have a low impedance, and the resistance natwork 60 may be formed with the elongated serpentine configuration described previously and with a relatively narrow cross-section for a relatively high impedance. As discussed below, the important value of the resistor network is not the absolute resistance, but rather the ratio of resistances that is created at the top connection tab. In one embodiment, a similar resistive network is formed in the controller electronics 40 located within the controller housing 42.

An important aspect of the preferred resistive networks formed in the patch and controller is that resistive ratios, rather than absolute values determine compatibility of the patch and controller. It is envisioned that problems could arise in manufacturing and in application due to tolerances in the resistors being compared. For example, if the tolerance of the resistors permits a controller to supply power to an incompatible patch, the patient may be seriously harmed. In order to avoid the problems associated with absolute values, the preferred embodiment of the present invention uses resistive ratios formed by the resistive networks previously described.

When the patch is connected to the controller, the resistive network on the patch makes and electrically couples with the resistive network on the controller to form, together, a resistive bridge circuit. If the resistance ratios of the patch resistive network and controller resistive network, which define the bridge circuit, match, the differential voltage between the taps of the two networks will be substantially zero volts.

If the differential voltage is substantially equal to zero, the patch and controller are deemed to be compatible. If the differential voltage is anything other than approximately zero, the patch and controller are deemed incompatible. The differential voltage may be used inthis case as a signal to the controller to instruct the controller not to deliver the drug to the patient.

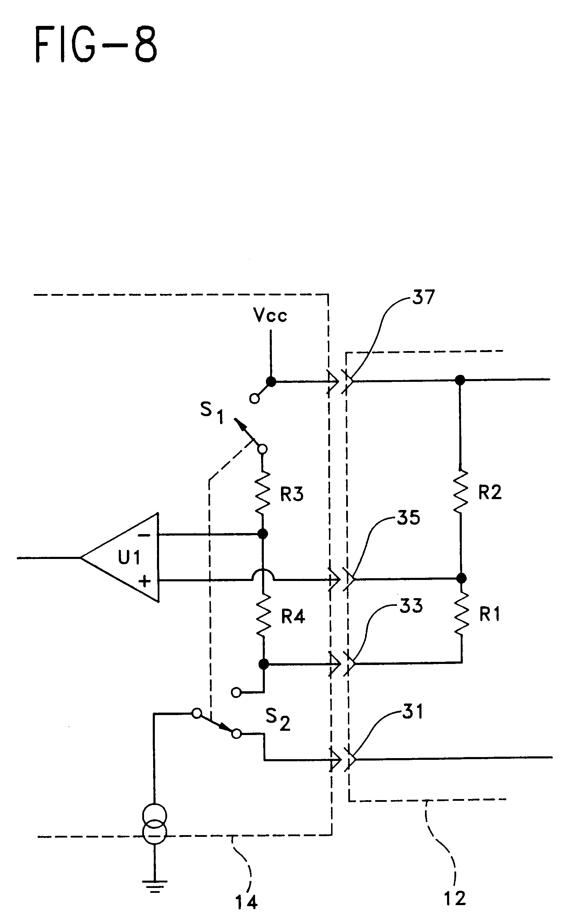

Referring to Figure 8, one embodiment of the compatibility determining means of the present invention is illustrated in a circuit schematic. The patch 12 is illustrated as including four connection tabs 31, 33, 35, and 37. Two tabs 31, 37 are connected to the patch electrodes. Tab 35 is the tap in the resistive network separating the resistor 62 into resistors R1 and R2. The other end of a resistor R2 is electrically connected to tab 33 and the patch anode. The opposite end of resistor R1 is coupled to tab 39. Controller 14 includes a similar resistor network illustrated by resistors R3 and R4. The opposite end of resistor R3 from the tap is connected to switch S1 for selective coupling to the controller power source Vcc which is coupled to the patch anode at tab 33. The opposite end of resistor R4 is coupled to patch tab 39 and switch S2. The controller also includes a differential operational amplifier having its inverted input coupled to the tap into the R3, R4 resistor bridge. The non-inverting input is coupled to the connection tab 35 of the patch. The controller also includes switches S1 and S2 to selectively direct current through the resistor bridge.

The resistor bridge identification means as illustrated in Figure 8 works as follows: the current that would normally flow through the anode to the cathode is redirected by switch arrangement S1 and S2. The ratio of resistors R2 and R1 on the patch are compared to the ratio of resistors R3 and R4 on the controller. When the current is directed through the resistor bridge circuit by switches S1 and S2, the differential operational amplifier is used to measure the offset of the bridge. This value can be determined by a microprocessor or by a simple comparator. If the offset is determined to be non-zero, the controller is deemed incompatible with the patch and will not operate.

In an alternative embodiment, the current passing through the patch resistive bridge circuit is measured by the controller electronics to determine a specific patch identification. The controller preferably includes a microprocessor to measure the voltage drop across each resistor of the resistive network of the patch and determine the ratio of the resistances comprising the network, and compare that ratio with a predetermined value stored in the microprocessor's memory. If the microprocessor determines that the measured voltage drop across the patch resistive network is compatible with the controller, the controller applies the proper current for the proper amount of time to treat the patient. Otherwise, if the patch and controller are deemed incompatible, the controller is disabled and no current is permitted to flow to the patch. The controller may also include means for indicating incompatibility with a patch such as a visual or audio indicator, e.g. an LED or an electronic buzzer, which may also be controlled by the microprocessor of the controller.

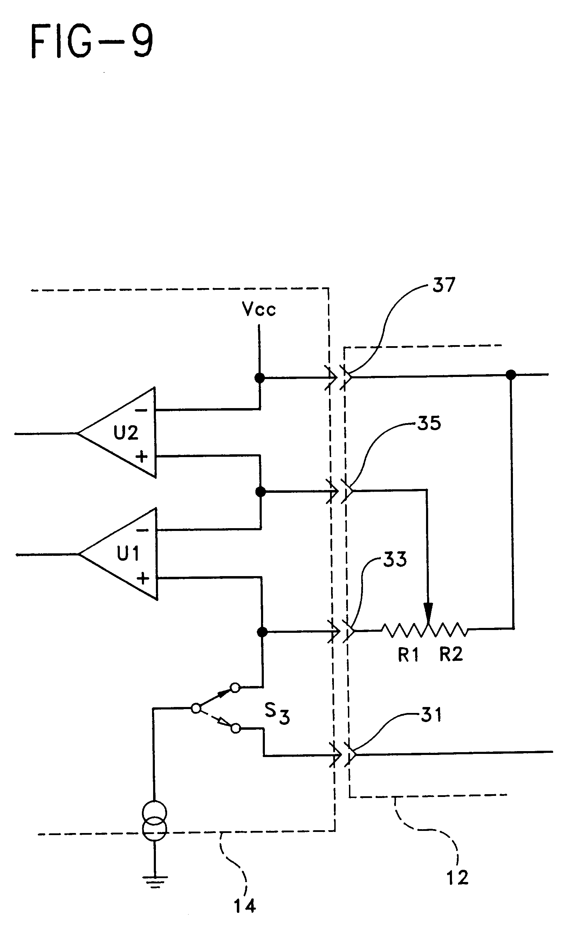

The embodiment described above is schematically illustrated in Figure 9. In Figure 9, the patch configuration remains the same as that illustrated in Figure 8. However, the controller electronics are slightly different. The controller includes four connection tabs to electrically connect the controller to the patch. The controller includes two differential operational amplifiers U2 and U3. A non-inverting input of U2 and an inverting input of U3 are coupled to the resistor network tap at tab 35. The non-inverting input of U3 is connected to the resistor network at tab 33. The inverting input of U2 is connected to the patch anode tab 37 as well as the controller power source Vcc. The controller includes switch S3 to direct the flow of current either through the resistors R1 and R2 of the patch resistor network or through the patch anode and cathode. The two differential operational amplifiers are used to measure the voltage drop across resistors R1 and R2 resulting in VR1 and VR2. The patch identification can be measured as the voltage VR2 divided by the voltage VR1 by a microprocessor having A/D converters. Based upon the patch identification, the compatibility of the patch and controller can be determined.

In yet another embodiment, the patch and controller identification may be in the form of an electronic serial number on the patch and means for reading the serial number in the controller. If the serial number is determined by the controller electronics to be a compatible patch, current is supplied to the patch. The serial number is preferably in the form of an integrated chip embedded in the patch. The controller is capable of being electrically coupled to the patch to read the serial number. The controller includes a microprocessor such that the controller may poll the patch to determine if the serial number is found in a look-up table in the microprocessor. If the serial number is not compatible with the particular controller, the controller will not be able to supply energy to the patch.

The present invention is also directed to the method of ensuring compatibility of a reusable controller with a patch to which it is connected. The method includes the steps of electrically comparing an identification of the patch and an identification of the controller to determine compatibility of the patch and controller and permitting current to flow from the controller to the patch only if the patch and controller are determined to have compatible identifications. Accordingly, the present invention provides an important safety feature of iontophoretic drug delivery devices which utilize reusable controllers.

Although the illustrative embodiments of the present invention have been described herein with reference to the accompanying drawings, it is to be understood that the invention is not limited to those precise embodiments, and that various other changes and modifications may be effected therein by one skilled in the art without departing from the scope of the invention.

## Claims

1. An iontophoretic drug delivery device which includes a medicament-containing disposable patch (12) removably attachable to the skin of the patient for transdermal delivery of an ionized medicament and a reusable controller (14) removably, electrically connectable to said patch (12), the controller providing sufficient energy to said patch (12) to drive the ionized medicament into the skin of a patient, characterised in that the controller (14) includes means (40) for detecting a number of times the controller (14) is used.

2. An iontophoretic drug delivery device according to claim 1, wherein the controller (14) includes clock means for timing the useful life of the controller (14), the controller (14) includes means for recording a date and time of usage for evaluating patient compliance in receiving medication as prescribed, the controller (14) includes a microprocessor (54) for storing a unique controller serial number to track said controller (14), the controller (14) includes means for detecting that a supply of the ionized medicament has been exhausted, the controller (14) includes means for rendering said controller (14) unusable when said detecting means determines that said supply of ionized medicament has been exhausted, and the controller (14) includes compatibility means for determining whether the reusable controller (14) is compatible with the medicament containing patch (12), the compatibility means preventing the controller (14) from supplying power to said patch (12) if said controller (14) and patch (12) are deemed incompatible.

3. An iontophoretic drug delivery device according to Claim 1, wherein said detecting means includes a microprocessor (54) which counts the number of applications of said controller (14).

4. An iontophoretic drug delivery device according to claim 3, wherein the microprocessor (54) includes disabling means which renders the controller (14) unusable when a specific number of applications have been sensed.

5. An iontophoretic drug delivery according to claim 1, wherein the controller (14) further includes clock means for timing a useful life of the controller (14).

6. An iontophoretic drug delivery device according to claim 5, wherein the clock means comprises a microprocessor (54) and wherein said microprocessor renders said controller (14) unusable when a specified period of time has elapsed.

7. An iontophoretic drug delivery device according to claim 1, wherein the controller (14) further includes means for recording a date and time of usage for evaluating patient compliance in receiving medication as prescribed.

8. An iontophoretic drug delivery device according to claim 1, wherein the controller (14) includes a microprocessor (54) having read only memory (45) with a unique serial number stored therein for tracking said controller (14).

9. An iontophoretic drug delivery device according to claim 1, wherein the controller (14) includes a microprocessor (54) and an LED (38) electrically connected to said microprocessor, said LED capable of being a transmitter for transmitting information obtained and stored in said microprocessor, and a photo receiver for receiving information to instruct said microprocessor.

10. An iontophoretic drug delivery device according to claim 1, wherein the controller (14) further includes means for detecting that a supply of medicament on said patch (12) has been exhausted and means for rendering said controller (14) unusable when said detecting means detects that said supply of medicament has been exhausted.

11. An iontophoretic drug delivery device according to claim 1, wherein the reusable controller (14) has a power source (5), and the patch (12) for attaching to the skin of a patient includes an anode (28) and a cathode (30), one of the said anode and said cathode including a reservoir (26) containing an ionizable substance for transcutaneous delivery to the patient, said patch (12) being removably, electrically connectable to said controller (14), and wherein the controller (14) further includes means for determining whether the controller (14) is compatible with the patch (12), the determining means preventing power from the controller (14) from being applied to said patch (12) if said controller (14) and patch (12) are deemed incompatible.

12. An iontophoretic drug delivery device according to claim 11, wherein the determining means comprises a resistor network in said controller (14) and a resistor network in said patch (12), said controller (14) including means for comparing resistive ratios of said controller (14) and patch networks.

13. An iontophoretic drug delivery device according to claim 12, wherein the controller (14) further includes means for disabling said controller (14) when the resistive ratios of said controller (14) and said patch (12) are determined to be incompatible.

14. An iontophoretic drug delivery device according to claim 11, wherein the determining means includes a serial number located on said patch (12), and said controller (14) includes means for reading said serial number.

15. An iontophoretic drug delivery device according to claim 11, wherein the determining means includes a bridge resistor network in said patch (12) and means in said controller (14) for measuring and calculating a voltage drop across said patch resistor bridge to determine a patch identification.

16. An iontophoretic drug delivery device according to claim 15, wherein the means for measuring and calculating said voltage drop comprises a pair of differential operation amplifiers having inputs coupled to said resistor bridge and a microprocessor (54) electrically connected to the outputs of said differential amplifiers.

17. An iontophoretic drug delivery device according to claim 1, wherein said patch (12) includes an anode (28) and a cathode (30), one of said anode and cathode including a reservoir (26) containing a said ionizable medicament; an electrically conductive lead (34) coupled to the cathode and an electrically conductive lead (32) coupled to said anode, wherein the patch (12) includes a resistive bridge (62) coupled to said cathode lead, and the controller (14) further includes a resistive network and means for comparing a resistive ratio of said controller network with a resistive ratio of said patch bridge circuit such that the controller (14) will permit current to flow only if the resistive ratio of said controller (14) and said resistive ratio of said patch (12) are deemed compatible.

## Patentansprüche

1. Vorrichtung zur iontophoretischen Verabreichung von Medikamenten, die ein das Medikament enthaltendes Einwegpflaster (12), das zur transdermalen Verabreichung eines ionisierten Medikaments abnehmbar auf die Haut des Patienten aufgebracht werden kann, und ein wiederverwendbares Steuergerät (14) einschließt, das abnehmbar elektrisch mit dem Pflaster (12) verbunden werden kann, um dem Pflaster (12) genügend Energie zuzuführen, um das ionisierte Medikament in die Haut eines Patienten einzuführen, dadurch gekennzeichnet, daß das Steuergerät (14) Mittel (40) zum Feststellen der Anzahl der Fälle, in denen das Steuergerät (14) eingesetzt wird, einschließt.

2. Vorrichtung zur iontophoretischen Verabreichung von Medikamenten nach Anspruch 1, bei der das Steuergerät (14) ein Taktmittel zur Zeitmessung der Nutzzeit des Steuergeräts (14) einschließt, das Steuergerät (14) Mittel zur Aufzeichnung eines Anwendungsdatums und einer ·zeit einschließt, um die Befolgung der Verabreichung des Medikaments an den Patienten nach Vorschrift zu bewerten, das Steuergerät (14) einen Mikroprozessor (54) zur Speicherung einer eindeutigen Seriennummer des Steuergeräts einschließt, um die Spur des Steuergeräts (14) zu verfolgen, das Steuergerät (14) Mittel für den Nachweis einschließt, daß ein Vorrat des ionisierten Medikaments verbraucht worden ist, das Steuergerät (14) Mittel einschließt, um das Steuergerät (14) unbrauchbar zu machen, wenn das Nachweismittel feststellt, daß der Vorrat des ionisierten Medikaments verbraucht worden ist, und das Steuergerät (14) Kompatibilitätsmittel einschließt, um festzustellen, ob das wiederverwendbare Steuergerät (14) mit dem medikamentenhaltigen Pflaster (12) kompatibel ist, wobei das Kompatibilitätsmittel verhindert, daß das Steuergerät (14) dem Pflaster (12) Energie zuführt, wenn das Steuergerät (14) und das Pflaster (12) als inkompatibel betrachtet werden.

3. Vorrichtung zur iontophoretischen Verabreichung von Medikamenten nach Anspruch 1, bei der das Nachweismittel einen Mikroprozessor (54) einschließt, der die Anzahl der Anwendungen des Steuergeräts (14) zählt.

4. Vorrichtung zur iontophoretischen Verabreichung von Medikamenten nach Anspruch 3, bei welcher der Mikroprozessor (54) Sperrmittel einschließt, die das Steuergerät (14) unbrauchbar machen, wenn eine bestimmte Anzahl von Anwendungen festgestellt worden ist.

5. Vorrichtung zur iontophoretischen Verabreichung von Medikamenten nach Anspruch 1, bei der das Steuergerät (14) außerdem ein Taktmittel zur Zeitmessung einer Nutzzeit des Steuergeräts (14) einschließt.

6. Vorrichtung zur iontophoretischen Verabreichung von Medikamenten nach Anspruch 5, bei der das Taktmittel einen Mikroprozessor (54) umfaßt und bei welcher der Mikroprozessor das Steuergerät (14) unbrauchbar macht, wenn eine bestimmte Zeitspanne verstrichen ist.

7. Vorrichtung zur iontophoretischen Verabreichung von Medikamenten nach Anspruch 1, bei der das Steuergerät (14) außerdem Mittel zur Aufzeichnung eines Anwendungsdatums und einer ·zeit einschließt, um die Befolgung der Verabreichung des Medikaments an den Patienten nach Vorschrift zu bewerten.

8. Vorrichtung zur iontophoretischen Verabreichung von Medikamenten nach Anspruch 1, bei der das Steuergerät (14) einen Mikroprozessor (54) einschließt, der einen Festspeicher (45) mit einer darin gespeicherten eindeutigen Seriennummer hat, um die Spur des Steuergeräts (14) zu verfolgen.

9. Vorrichtung zur iontophoretischen Verabreichung von Medikamenten nach Anspruch 1, bei der das Steuergerät (14) folgendes einschließt: einen Mikroprozessor (54) und eine Lumineszenzdiode (38), die elektrisch mit dem Mikroprozessor verbunden ist, wobei die Lumineszenzdiode ein Sender zur Übertragung von Informationen sein kann, die in dem Mikroprozessor ermittelt und gespeichert worden sind, und einen Fotoreceiver zum Empfang von Informationen als Instruktionen für den Mikroprozessor.

10. Vorrichtung zur iontophoretischen Verabreichung von Medikamenten nach Anspruch 1, bei der das Steuergerät (14) außerdem Mittel für den Nachweis, daß ein Vorrat des Medikaments auf dem Pflaster (12) verbraucht worden ist, und Mittel einschließt, um das Steuergerät (14) unbrauchbar zu machen, wenn das Nachweismittel feststellt, daß der Vorrat des Medikaments verbraucht worden ist.

11. Vorrichtung zur iontophoretischen Verabreichung von Medikamenten nach Anspruch 1, bei der das wiederverwendbare Steuergerät (14) eine Energiequelle (5) hat und das Pflaster (12) zum Aufbringen auf der Haut des Patienten eine Anode (28) und eine Kathode (30) einschließt, wobei eines der Elemente Anode und Kathode ein Reservoir (26) einschließt, das eine ionisierbare Substanz für die transkutane Verabreichung an den Patienten enthält, wobei das Pflaster (12) abnehmbar elektrisch mit dem Steuergerät (14) verbunden werden kann, und bei der das Steuergerät (14) außerdem Mittel einschließt, um festzustellen, ob das Steuergerät (14) mit dem Pflaster (12) kompatibel ist, wobei das Mittel zum Feststellen verhindert, daß dem Pflaster (12) von dem Steuergerät (14) Energie zugeführt wird, wenn das Steuergerät (14) und das Pflaster (12) als inkompatibel betrachtet werden.

12. Vorrichtung zur iontophoretischen Verabreichung von Medikamenten nach Anspruch 11, bei der das Mittel zum Feststellen ein Widerstandsnetz in dem Steuergerät (14) und ein Widerstandsnetz in dem Pflaster (12) umfaßt, wobei das Steuergerät (14) Mittel für den Vergleich der Widerstandsverhältnisse der Netze des Steuergeräts (14) und des Pflasters einschließt.

13. Vorrichtung zur iontophoretischen Verabreichung von Medikamenten nach Anspruch 12, bei der das Steuergerät (14) außerdem Mittel zum Sperren des Steuergeräts (14) einschließt, wenn die Widerstandsverhältnisse des Steuergeräts (14) und des Pflasters (12) als inkompatibel festgestellt werden.

14. Vorrichtung zur iontophoretischen Verabreichung von Medikamenten nach Anspruch 11, bei der das Mittel zum Feststellen eine Seriennummer einschließt, die sich auf dem Pflaster (12) befindet, und das Steuergerät (14) Mittel zum Lesen der Seriennummer einschließt.

15. Vorrichtung zur iontophoretischen Verabreichung von Medikamenten nach Anspruch 11, bei der das Mittel zum Feststellen ein Widerstandsbrückennetz in dem Pflaster und in dem Steuergerät (14) Mittel zum Messen und Berechnen eines Spannungsabfalls an der Widerstandsbrücke des Pflasters einschließt, um eine Pflasteridentifikation vorzunehmen.

16. Vorrichtung zur iontophoretischen Verabreichung von Medikamenten nach Anspruch 15, bei der das Mittel zum Messen und Berechnen des Spannungsabfalls ein Paar Differentialoperationsverstärker, die mit der Widerstandsbrücke gekoppelte Eingänge haben, und einen Mikroprozessor (54) umfaßt, der elektrisch mit den Ausgängen der Differentialverstärker verbunden ist.

17. Vorrichtung zur iontophoretischen Verabreichung von Medikamenten nach Anspruch 1, bei der das Pflaster (12) folgendes einschließt: eine Anode (28) und eine Kathode (30), wobei eines der Elemente Anode und Kathode ein Reservoir (26) einschließt, welches das ionisierbare Medikament enthält, eine elektrisch leitende Leitung (34), die mit der Kathode gekoppelt ist, und eine elektrisch leitende Leitung (32), die mit der Anode gekoppelt ist, bei der das Pflaster (12) eine Widerstandsbrücke (62) einschließt, die mit der Kathodenleitung gekoppelt ist, und das Steuergerat (14) außerdem ein Widerstandsnetz und Mittel zum Vergleich eines Widerstandsverhältnisses des Steuergerätnetzes mit einem Widerstandsverhaltnis der Pflaster-Brückenschaltung einschließt, derartig, daß das Steuergerät (14) den Stromfluß nur zuläßt, wenn das Widerstandsverhältnis des Steuergeräts (14) und das Widerstandsverhältnis des Pflasters (12) als kompatibel betrachtet werden.

## Revendications

1. Dispositif d'administration d'un médicament par ionophorèse englobant un timbre à usage unique contenant un médicament (12), fixé de manière amovible à la peau du patient en vue d'une administration transdermique d'un médicament ionisé, et un dispositif de commande réutilisable (14), pouvant être connecté électriquement et de manière amovible audit timbre (12), le dispositif de commande fournissant suffisamment d'énergie audit timbre (12) pour assurer l'introduction du médicament ionisé dans la peau d'un patient, caractérisé en ce que le dispositif de commande (14) englobe un moyen (40) pour détecter le nombre d'utilisations du dispositif de commande (14).

2. Dispositif d'administration d'un médicament par ionophorèse selon la revendication 1, dans lequel le dispositif de commande (14) englobe un moyen d'horloge pour mesurer la durée de vie utile du dispositif de commande (14), le dispositif de commande (14) englobant un moyen pour enregistrer une date et une durée d'utilisation pour évaluer l'adaptabilité d'un patient à la réception du médicament prescrit, le dispositif de commande (14) englobant un microprocesseur (54) destiné à enregistrer un numéro de série unique du dispositif de commande, pour assurer le suivi dudit dispositif de commande (14), le dispositif de commande (14) englobant un moyen pour détecter qu'une réserve d'un médicament ionisé est épuisée, le dispositif de commande (14) englobant un moyen pour rendre ledit dispositif de commande (14) inutilisable lorsque ledit moyen de détection détermine que ladite réserve du médicament ionisé est épuisée, et le dispositif de commande (14) englobant un moyen de compatibilité pour déterminer si le dispositif de commande réutilisable (14) est compatible avec le timbre contenant le médicament (12), le moyen de détermination de la compatibilité empêchant le dispositif de commande (14) d'alimenter en énergie ledit timbre (12) lorsque ledit dispositif de commande (14) et le timbre (12) sont déterminés comme étant incompatibles.

3. Dispositif d'administration d'un médicament par ionophorèse selon la revendication 1, dans lequel ledit moyen de détection englobe un microprocesseur (54) comptant le nombre d'applications dudit dispositif de commande (14).

4. Dispositif d'administration d'un médicament par ionophorèse selon la revendication 3, dans lequel le microprocesseur (54) englobe un moyen d'invalidation rendant le dispositif de commande (14) inutilisable après la détection d'un nombre d'applications spécifique.

5. Dispositif d'administration d'un médicament par ionophorèse selon la revendication 1, dans lequel le dispositif de commande (14) englobe en outre un moyen d'horloge pour mesurer une durée de vie utile du dispositif de commande (14).

6. Dispositif d'administration d'un médicament par ionophorèse selon la revendication 5, dans lequel le moyen d'horloge comprend un microprocesseur (54), ledit microprocesseur rendant ledit dispositif de commande (14) inutilisable après l'écoulement d'une période de temps spécifiée.

7. Dispositif d'administration d'un médicament par ionophorèse selon la revendication 1, dans lequel le dispositif de commande (14) englobe en outre un moyen pour enregistrer une date et une durée d'utilisation, pour évaluer l'adaptabilité du patient à la réception du médicament prescrit.

8. Dispositif d'administration d'un médicament par ionophorèse selon la revendication 1, dans lequel le dispositif de commande (14) englobe un microprocesseur (54) comportant une mémoire morte (45), un numéro de série unique y étant enregistré en vue du suivi dudit dispositif de commande (14).

9. Dispositif d'administration d'un médicament par ionophorèse selon la revendication 1, dans lequel le dispositif de commande (14) englobe un microprocesseur (54) et une DEL (38), connectée électriquement audit microprocesseur, ladite DEL étant capable de faire fonction d'émetteur pour transmettre des informations obtenues et enregistrées dans ledit microprocesseur, et de récepteur optique pour recevoir des informations en vue d'instruire ledit microprocesseur.

10. Dispositif d'administration d'un médicament par ionophorèse selon la revendication 1, dans lequel le dispositif de commande (14) englobe en outre un moyen pour détecter qu'une réserve du médicament contenu sur ledit timbre (12) est épuisée et un moyen pour rendre ledit dispositif de commande (14) inutilisable lorsque ledit moyen de détection détecte que ladite réserve de médicament est épuisée.

11. Dispositif d'administration d'un médicament par ionophorèse selon la revendication 1, dans lequel le dispositif de commande réutilisable (14) comporte une source d'énergie (5), le timbre (12) destiné à être fixé à la peau d'un patient englobant une anode (28) et une cathode (30), l'une de ladite anode ou de ladite cathode englobant un réservoir (26) contenant une substance ionisable en vue d'une administration transcutanée au patient, ledit timbre (12) pouvant être connecté électriquement et de façon amovible audit dispositif de commande (14), le dispositif de commande (14) englobant en outre un moyen pour déterminer si le dispositif de commande (14) est compatible avec le timbre (12), le moyen de détermination empêchant l'application de l'énergie du dispositif de commande (14) audit timbre (12) lorsque ledit dispositif de commande (14) et ledit timbre (12) sont déterminés comme étant incompatibles.

12. Dispositif d'administration d'un médicament par ionophorèse selon la revendication 11, dans lequel le moyen de détermination comprend un réseau de résistance dans ledit dispositif de commande (14) et un réseau de résistance dans ledit timbre (12), ledit dispositif de commande (14) englobant un moyen pour comparer les rapports de résistance des réseaux dudit dispositif de commande (14) et dudit timbre.

13. Dispositif d'administration d'un médicament par ionophorèse selon la revendication 12, dans lequel le dispositif de commande (14) englobe en outre un moyen pour invalider le dispositif de commande (14) lorsque les rapports de résistance dudit dispositif de commande (14) et dudit timbre (12) sont déterminés comme étant incompatibles.

14. Dispositif d'administration d'un médicament par ionophorèse selon la revendication 11, dans lequel le moyen de détermination englobe un numéro de série appliqué sur ledit timbre (12), ledit dispositif de commande (14) englobant un moyen pour lire ledit numéro de série.

15. Dispositif d'administration d'un médicament par ionophorèse selon la revendication 11, dans lequel le moyen de détermination englobe un réseau de pont de résistance dans ledit timbre (12) et un moyen dans ledit dispositif de commande (14) pour mesurer et calculer une chute de tension à travers ledit pont de résistance du timbre pour déterminer une identification du timbre.

16. Dispositif d'administration d'un médicament par ionophorèse selon la revendication 15, dans lequel le moyen de mesure et de calcul de ladite chute de tension comprend une paire d'amplificateurs opérationnels différentiels comportant des entrées couplées audit pont de résistance, et un microprocesseur (54), connecté électriquement aux sorties desdits amplificateurs différentiels.

17. Dispositif d'administration d'un médicament par ionophorèse selon la revendication 1, dans lequel ledit timbre (12) englobe une anode (28) et une cathode (30), l'une de ladite anode ou de ladite cathode englobant un réservoir (26) contenant ledit médicament ionisable, un fil électroconducteur (34) couplé à la cathode et un fil électroconducteur (32) couplé à ladite anode, le timbre (12) englobant un pont de résistance (62) couplé audit fil de la cathode, le dispositif de commande (14) englobant en outre un réseau de résistance et un moyen pour comparer un rapport de résistance dudit réseau du dispositif de commande avec un rapport de résistance dudit circuit du pont du timbre, de sorte que le dispositif de commande (14) ne permet l'écoulement de courant que si le rapport de résistance dudit dispositif de commande (14) et ledit rapport de résistance dudit timbre (12) sont déterminés comme étant compatibles.
